# EUROPEAN PATENT SPECIFICATION

(11) **EP 3 973 934 B1**
(45) Date of publication and mention of the grant of the patent: **20.12.2023**
(21) Application number: 21198652.6
(22) Date of filing: 23.09.2021
(51) Int. Cl.: A61F 13/00, A61L 15/24, C08F 220/14, C08F 220/18, C08F 220/60, A01N 47/44, C09D 133/24, A01P 1/00, A61L 15/46, A61L 27/54, A61L 27/34, A61F 13/04

(54) **POLYCATIONIC POLYMER, COMPOSITION COMPRISING THE POLYCATIONIC POLYMER, AND PRODUCT, COMPRISING AN ACTIVE SURFACE, PROVIDED WITH THE POLYCATIONIC POLYMER**
POLYKATIONISCHES POLYMER, ZUSAMMENSETZUNG MIT DEM POLYKATIONISCHEN POLYMER UND PRODUKT MIT EINER AKTIVEN OBERFLÄCHE MIT DEM POLYKATIONISCHEN POLYMER
POLYMÈRE POLYCATIONIQUE, COMPOSITION COMPRENANT LE POLYMÈRE POLYCATIONIQUE, ET PRODUIT COMPRENANT UNE SURFACE ACTIVE DOTÉE DU POLYMÈRE POLYCATIONIQUE

(30) Priority: 25.09.2020 NL 2026546
(43) Date of publication of application: 30.03.2022
(73) Proprietor: Cobatt, 3881 BP Putten (NL)
(72) Inventor: BATTERINK, Henri, 3881 BP Putten (NL)
(74) Representative: Nederlandsch Octrooibureau

(56) References cited:
- CN-B- 106 893 054
- US-A1- 2020 009 182
- ANTOINE FR?RE ET AL: "Impact of the Structure of Biocompatible Aliphatic Polycarbonates on siRNA Transfection Ability", BIOMACROMOLECULES, vol. 16, no. 3, 9 March 2015 (2015-03-09), pages 769-779, XP055330501, US ISSN: 1525-7797, DOI: 10.1021/bm501676p
- H. SONG ET AL: "Synthesis and characterization of a new family of cationic amino acid-based poly(ester amide)s and their biological properties", JOURNAL OF APPLIED POLYMER SCIENCE, vol. 124, no. 5, 27 November 2011 (2011-11-27), pages 3840-3853, XP055417138, US ISSN: 0021-8995, DOI: 10.1002/app.35512
- GANG XU ET AL: "Arginine-Based Polymer Brush Coatings with Hydrolysis-Triggered Switchable Functionalities from Antimicrobial (Cationic) to Antifouling (Zwitterionic)", LANGMUIR, vol. 33, no. 27, 11 July 2017 (2017-07-11) , pages 6925-6936, XP055394077, US ISSN: 0743-7463, DOI: 10.1021/acs.langmuir.7b01000
- MAREIKE BARDTS ET AL: "Novel copolymers showing interactions of amidinium-carboxylate groups in water", JOURNAL OF POLYMER RESEARCH, KLUWER ACADEMIC PUBLISHERS-CONSULTANTS BUREAU, NL, vol. 18, no. 1, 24 February 2010 (2010-02-24), pages 151-155, XP019867643, ISSN: 1572-8935, DOI: 10.1007/S10965-010-9401-9

## Description

### FIELD OF THE INVENTION

The invention relates to a polycationic polymer. The invention further relates to a composition comprising the polycationic polymer, and a product comprising a functionalized surface provided with the polycationic polymer. The invention also relates to the polycationic polymer for use as a medicament and for use as a topical skin composition, such as a cream or ointment.

### BACKGROUND OF THE INVENTION

Microbes, such as bacteria, fungi and parasites, are living organisms. While some of these microbes may be beneficial, many of these are pathogenic (i.e. may cause infections of cells, e.g. human cells). Pathogenic microbes are the target of antimicrobial agents.

Antimicrobial polymers are known to be suitable for counteracting pathogenic microbes. These can be classified as passive polymers, which repel microbes, or as active polymers, which kill microbes. For both kinds of polymers, multiple examples are known.

A first known type of antimicrobial polymers with microbial activity is based on polymers which are functionalized with a positively-charged quaternary ammonium group. This group is able to interact with the negatively-charged cytoplasmic membrane of the microbe. As a consequence, the intracellular components will leak out of the cell, causing cell death. While these polymers may be satisfactory for some applications, these have a number of disadvantages. For instance, these polymers are typically not able to counteract a wide spectrum of microbes. Furthermore, the application of these polymers may lead mutations within the microbe, which may cause the microbe to become resistant, possibly decreasing the efficacy of the antimicrobial polymers.

Another type of polymers with microbial activity comprises the class of biocide-releasing polymers. In these polymers, the polymer is used as a carrier for an antimicrobial substance, such as silver. This substance is bound to the carrier such that it is able to be released therefrom by leaching, killing the microbe. As a consequence, these leaching polymers have to comply with much more strict requirements than non-leaching antimicrobial polymers.

Furthermore, quaternary ammonium compounds are yet another type of known antimicrobial agents. While these compounds are effective against gram-positive bacteria, their efficacy against gram-negative bacteria is much less. CN106893054B discloses cationic polymers gene carriers, comprising a N-3-guanidine propyl methacrylamide repeating unit, which can load a large amount of gene drags and have better cell membrane penetration ability and targeting performance. Langmuir 2017, 33, 6925-6936 discloses Arginine polymer based coatings with switchable properties developed on glass slides (GS) to demonstrate the smart transition from antimicrobial (cationic) to fouling-resistant (zwitterionic) surfaces. L-Arginine methyl ester-methacryloylamide (Arg-Est) and L-arginine-methacryloylamide (Arg-Me) polymer brushes were grafted from the GS surface via surface-initiated reversible addition-fragmentation chain-transfer (SI-RAFT) polymerization. J. Polymer Res (2011) 18: 151-155 discloses a copolymer of methyl-5-guanidino-2-methacrylamidopentanoate with a water soluble co-monomer N,N-dimethylacrylamide having a dispersity of 2,7.

### SUMMARY OF THE INVENTION

It is an object of the present invention to provide a polymer which is able to counteract a wider spectrum of microbes than the previously described antimicrobial polymers. It is another object of the present invention to provide a polymer reducing the tendency of microbes to mutate as a consequence of its use. It is another object of the present invention to provide a polymer of the type with a limited or no tendency to leach out under operational circumstances.

This object is achieved with a polycationic polymer, comprising a cationic repeating unit C, comprising a positively-charged protonated guanidine group, and a negatively-charged counterion, balancing the positive charge of this guanidine group, wherein the dispersity (M_{w}/Mₙ) of the polymer is 1,5 or higher. Dispersity (Mw/Mn) of the polymer is determined using gel permeation chromatography (GPC). For determining the dispersity, a sample of the polymer is weighed and dissolved in tetrahydrofuran (THF), and stirred for 2 hours. Subsequently, a determined amount of the dissolved polymer sample is injected onto a Shim-pack-806 GPC column (Shimadzu; exclusion limit is 4 x 107) in a Shimadzu GPC system comprising an LC-20AD pump (Shimadzu) and an RID-10A detector (Shimadzu), for determining the dispersity.

Guanidine groups are able to attract microbes, because of their positive charge. The presence of a counterion, balances the positive charge of the protonated guanidine group and thereby decreases or even prevents the repeating unit C from having no zwitterionic character, implying that the repeating unit can remain positively charged making it possible for the protonated guanidine group to be present as a resonance hybrid, i.e. in which the location of the double bond changes, which is believed to make the guanidine group to be able to "vibrate", which makes it capable of destroying cell membranes. By providing a polycationic polymer which does not only have this counterion and thus does have no negative charges within the polycationic polymer, and also has a relatively high dispersity, it is surprisingly found that the polycationic polymer is highly effective against a wide spectrum of microbes. For instance, the polycationic polymer according to the invention is with a surprisingly good efficacy against both gram-positive bacteria, such as but not limited to *Staphylococcus aureus,* and gram-negative bacteria, such as but not limited to *Escherichia coli.*

Preferably, the polycationic polymer comprises one or more than one counterion, for instance one counterion per cationic repeating unit C and thus, in the following, the reference to "the counterion" may also be interpreted as "the counterions". The one or more than one counterion preferably balances the positive charge of all of the guanidine groups in the polycationic polymer, increasing the efficacy of the polycationic polymer.

The synthesis of the polymer may be achieved in any suitable way. Examples are the synthesis of a polymeric backbone, which is functionalized with cationic structural units to obtain repeating units comprising a guanidine group after the synthesis of the backbone and/or by polymerization of a monomer comprising a guanidine group, preferably by radical polymerization. In such a case, obtaining a polycationic polymer with a dispersity according to the invention typically requires the use of free radical polymerization, in contrast to living polymerization such as reversible addition-fragmentation chain-transfer (RAFT) polymerization, which is expected to result in a polymer with a lower dispersity of approximately 1,0. The guanidine group may be positively-charged and/or protonated and/or bonded to a negatively-charged counterion as it is being functionalized or polymerized, respectively, or may be modified therefore afterwards.

The dispersity of the polymer according to the invention may preferably be between 1,5 and 6,0, and more preferably be between 2,0 and 5,5, even more preferably be between 2,5 and 5,0, and may still more preferably be between 3,0 and 4,0.

In an embodiment of the polycationic polymer according to the invention, the counterion is inorganic and is preferably selected from a chloride, bromide, iodide, fluoride, and more preferably is chloride.

Such counterions are preferred, since these are able to balance the positive charge of the cationic repeating unit C, while at the same time providing adequate solubility of the polycationic polymer in aqueous carrier liquids.

In an embodiment of the polycationic polymer according to the invention, the counterion is bound to the hydrogen by which the guanidine group is protonated.

Typical agents protonating the guanidine group while balancing the positive charge by providing the counterion thus comprise hydrogen chloride, hydrogen bromide, hydrogen iodide or hydrogen fluoride, and preferably comprise or are hydrogen chloride.

In an embodiment of the polycationic polymer according to the invention, said guanidine group is a terminal group.

The provision of the guanidine group as a terminal group in the chain positions the guanidine group at a location relatively spaced apart from the rest of the polymer, which may reduce charge compensation within the cationic repeating unit C, thereby increasing its efficacy.

In an embodiment of the polycationic polymer according to the invention, said guanidine group has the following structure: 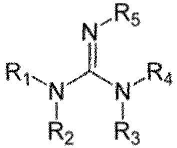
wherein one of R₁, R₂, R₃, R₄ and R₅ is an alkyl linking group, linking the guanidine group to the polymer backbone, and
wherein the others of R₁, R₂, R₃, R₄ and R₅ is a terminal group, preferably H.

By consequently linking the guanidine group to the polymer backbone with just one alkyl linking group, while the other groups of R₁, R₂, R₃, R₄ and R₅ are terminal groups, the guanidine group will more readily be able to act on microbes, since it experiences less hindrance compared to other envisageable structures.

While the others of R₁, R₂, R₃, R₄ and R₅ may be any suitable group, at least one of these and preferably all of these comprises a non-conjugated bonding to the nitrogen of the guanidine group. More preferably, at least one of these, and preferably all of these are preferably each H, to obtain an isolated group, for enhanced antimicrobial efficacy.

The alkyl linking group may have a relatively short length, i.e. between from C₁ to C₅, but may also have a longer length, e.g. between from C₆ to C₁₀ and/or between from C₁₁ to C₁₅. Choosing such a relatively longer chain length may enhance their antimicrobial activity.

In an embodiment of the polycationic polymer according to the invention, the cationic repeating unit further comprises an alkyl ester group.

As mentioned in general with respect to the polycationic polymer, it is important for the cationic repeating unit C to not have a zwitterionic characteristic. When the cationic repeating unit comprises an acid group, one preferred way of obtaining this comprises the use of an alcohol for converting said acid group to an alkyl ester group, said ester being selected from a methyl ester, an ethyl ester, a butyl ester and a propyl ester, preferably a methyl ester or an ethyl ester.

In an embodiment of the polycationic polymer according to the invention, the cationic repeating unit is formed from a monomer having a polymerizable group selected from an acrylate and a methacrylate.

As mentioned, one way of obtaining the polycationic polymer according to the invention involves polymerization of a monomer comprising a guanidine group, such as by radical polymerization. Selecting the polymerizable group for obtaining such a polymer from an acrylate and a methacrylate is advantageous, since these groups are both reactive for polymerization thereof, and have the possibility of carrying the guanidine group.

In an embodiment of the polycationic polymer according to the invention, the monomer has the following structure: 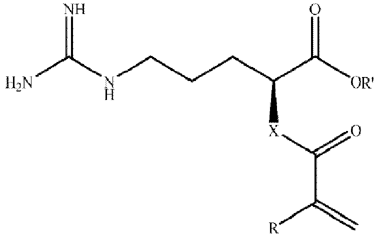
wherein X is NH, NH-alkyl-O, NH-alkyl-NR, NHCHzCH(OH)CHzO or NH-alkyl;
wherein each R is independently H or CH₃, and
wherein R' is alkyl.

Arginine is an amino acid which occurs in nature. Using arginine in the monomer for obtaining the polymer is advantageous since this will facilitate synthesis. Furthermore, this may increase the biocompatibility of the polymer compared to other possible polycationic polymers. The N in each of the possible choices for X in this structure is part of arginine, whereas the (other) N- or O- group at the opposite end is coupled to the acrylic group of this structure.

In an embodiment of the polycationic polymer according to the invention, R' is selected from methyl, ethyl, butyl and propyl and preferably is selected from methyl and ethyl.

Selecting R' from methyl, ethyl, butyl and propyl is a way of obtaining a polycationic polymer having cationic repeating units C which is not zwitterionic which makes it harder or impossible for a microbe to ingest the polymer, reducing the chance of mutations in the microbe. Methyl and ethyl are preferred, since this increases the solubility in water compared to longer alkyl chains. As mentioned, such a structure may for instance be obtained by esterification of a carboxyl moiety of the cationic repeating unit with an alcohol, e.g. methanol, ethanol, butanol or propanol.

Preferably, a monomer from which the polycationic polymer is formed has the following structure: 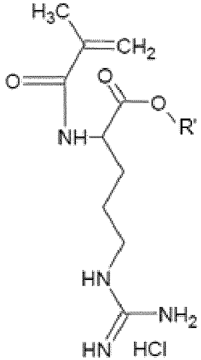 with R' being either methyl or ethyl.

In an embodiment of the polycationic polymer according to the invention, at least one cationic repeating unit comprises at least one further guanidine group, wherein said further guanidine group is linked to said guanidine group via at least one saturated C-C bond.

The provision of a further cationic guanidine group in at least one cationic repeating unit C ensures that at least one and preferably more or all of the guanidine groups keeps its ability to vibrate, necessary for its antimicrobial activity as described in the foregoing, thereby increasing the efficiency of the polycationic polymer in acting against microbes. However, in such a case, this further guanidine group is linked to the other by at least one saturated C-C bond for spacing these groups from each other. Two guanidine groups which are directly linked to each other may interact with each other in a way limiting its ability to act against microbes.

In an embodiment of the polycationic polymer according to the invention, the polycationic polymer comprises one or more than one further repeating unit, formed from a comonomer comprising a polymerizable group.

By including a further repeating unit, it is possible to further tune the properties of the polycationic polymer, such as for instance its solubility in aqueous carrier liquids such as water. The comonomer is coupled to the polycationic polymer by the polymerizable group.

In an embodiment of the polycationic polymer according to the invention, the polymerizable group of said further comonomer is selected from an acrylate and a methacrylate.

Selecting the polymerizable group from the further comonomer from an acrylate and a methacrylate increases its compatibility with the other polymerizable groups. More preferably, the polymerizable group of the comonomer and the monomer with the guanidine group is the same.

In an embodiment of the polycationic polymer according to the invention, said at least one comonomer is a crosslinkable monomer.

The provision of crosslinks in the polycationic polymer makes it harder for the polymer to dissolve and thus is stable, which is advantageous for application of the polycationic polymer in coatings, in particular antimicrobial coatings for prolonged use, such as on implants. Crosslinks may for instance be created under the influence of light, such as UV-light, with a suitable photo-initiator.

In an embodiment of the polycationic polymer according to the invention, the crosslinkable monomer is selected from benzophenone methacrylate (BPMA), benzophenone acrylate (BPA), a bifunctional acrylate or methacrylate, a trifunctional acrylate or methacrylate and a glycidyl acrylate or methacrylate.

The above-mentioned crosslinkable monomers are expected to result in a polycationic polymer which in use is easily applied and requires vigorous washing for removal, in order to avoid unintended removal.

An embodiment is the polycationic polymer of the invention, comprising a cationic repeating unit C, comprising a positively-charged protonated guanidine group, and a negatively-charged counterion, balancing the positive charge of this guanidine group, wherein the dispersity (M_{w}/Mₙ) of the polymer is 1,5 or higher, wherein the polycationic polymer comprises one or more than one further repeating unit, formed from a comonomer comprising a polymerizable group, wherein said at least one comonomer is a crosslinkable monomer, wherein the crosslinkable monomer is selected from benzophenone methacrylate (BPMA), benzophenone acrylate (BPA), a bifunctional acrylate or methacrylate, a trifunctional acrylate or methacrylate and a glycidyl acrylate or methacrylate. Dispersity (Mw/Mn) of the polymer is determined using gel permeation chromatography (GPC). For determining the dispersity, a sample of the polymer is weighed and dissolved in tetrahydrofuran (THF), and stirred for 2 hours. Subsequently, a determined amount of the dissolved polymer sample is injected onto a Shim-pack-806 GPC column (Shimadzu; exclusion limit is 4 x 107) in a Shimadzu GPC system comprising an LC-20AD pump (Shimadzu) and an RID-10A detector (Shimadzu), for determining the dispersity.

In an embodiment of the polycationic polymer according to the invention, the polymer is at least partly crosslinked.

In an embodiment of the polycationic polymer according to the invention, the average degree of crosslinking of the polymer is between 0,05 and 3 mole percent.

The degree of crosslinking preferably is within this range. At a degree of crosslinking below 0,05 mole percent, the degree is too low to observe a difference in characteristics compared to polymer which is uncrosslinked. At a degree of crosslinking higher than 3 mole percent, the material will become difficult to process, because of its toughness.

In an embodiment of the polycationic polymer according to the invention, at least one further comonomer is selected from:
a) at least one of benzyl methacrylate, butyl methacrylate, isobutyl acrylate, methyl methacrylate, ethyl methacrylate, propyl methacrylate, hexyl methacrylate, 2-ethylhexyl methacrylate, octyl methacrylate, lauryl methacrylate, stearyl methacrylate, p-tolyl methacrylate, sorbyl methacrylate, isobornyl methacrylate, benzyl acrylate, butyl acrylate, isobutyl acrylate, methyl acrylate, ethyl acrylate, propyl acrylate, hexyl acrylate, 2-ethylhexyl acrylate, octyl acrylate, lauryl acrylate, stearyl acrylate, p-tolyl acrylate, sorbyl acrylate isobornyl acrylate, and/or
b) at least one of tri(ethylene glycol) methyl ether acrylate, 2-(2ethoxyethoxy)ethyl acrylate (EOEOEA), ethylene glycol methyl ether acrylate, 2-ethoxyethyl acrylate, di(ethylene glycol) methyl ether acrylate, hydroxyethyl acrylate, methoxypoly(ethyleneglycol) acrylate, ethylene glycol methyl ether methacrylate, 2-ethoxyethyl methacrylate, di(ethylene glycol) methyl ether methacrylate, tri(ethylene glycol) methyl ether methacrylate, 2-(2ethoxyethoxy)ethyl methacrylate (EOEOEMA), hydroxyethyl methacrylate, methoxypoly(ethyleneglycol) methacrylate
and mixtures thereof.

Comonomers mentioned under a) may be preferred for their hydrophobic character, whereas comonomers mentioned under b) may be preferred for their hydrophilic character. The comonomers used may consequently be chosen based on the characteristics of the composition in which the polycationic polymer is being applied, e.g. based on the carrier liquid in which the polycationic polymer is to be applied.

When selecting methoxypoly(ethyleneglycol) methacrylate, the polyethylene glycol block in this monomer preferably has a M_{w} of 1.000 grams per mole or lower, such as 350 grams per mole or 500 grams per mole.

In an embodiment of the polycationic polymer according to the invention, the repeating unit C content of the polymer is between 1 and 50 percent by weight of the polymer, more preferably between 2 and 25 percent by weight of the polymer, most preferably between 5 and 15 percent by weight of the polymer, in particular approximately 10 percent by weight of the polymer.

Such repeating unit contents allow the polycationic polymer to perform its function of acting against microbes, while providing sufficient possibilities for adapting the structure of the polycationic polymer for adapting the properties, such as water solubility and/or reducing the cost of the polycationic polymer.

In an embodiment of the polycationic polymer according to the invention, the weight average molecular weight (M_{w}) of the polymer is between 10.000 and 1.000.000 Da, more preferably between 10.000 and 100.000 Da. The weight average molecular weight (Mw) of the polymer is determined using GPC. For determining the Mw, a sample of the polymer is weighed and dissolved in tetrahydrofuran (THF), and stirred for 2 hours. Subsequently, a determined amount of the dissolved polymer sample is injected onto a Shim-pack-806 GPC column (Shimadzu; exclusion limit is 4 x 107) in a Shimadzu GPC system comprising an LC-20AD pump (Shimadzu) and an RID-10A detector (Shimadzu), for determining the Mw.

A polycationic polymer with a weight average molecular weight within this range, and preferably within the preferred range, is expected to be able to rupture the cell membrane of a microbe, such as bacteria, while not being able to be ingested by it. This reduces the risks of mutations within the microbe.

The polycationic polymer according to the invention may for instance be formed from a mixture of L-arginine hydrochloride methyl ester methacrylamide, benzophenone methacrylate, 2-ethylhexyl acrylate and butyl acrylate monomers.

A crosslinkable polycationic polymer according to the invention may for instance be formed from L-arginine hydrochloride methyl ester methacrylamide, methoxypoly(ethyleneglycol) methacrylate with a molecular weight of the PEG block of 350 grams per mole, methyl methacrylate and butyl acrylate monomers.

In an embodiment of the polycationic polymer according to the invention, the polycationic polymer is for use as a medicament.

The polycationic polymer has been shown to be able to act against microbes, and may as a consequence be used for treating microbial infections, such as impetigo, cold sores, athlete's foot and onychauxis. Furthermore, its capability of slowing down enzymatic activity of enzymes secreted by mosquitos when biting, makes the polycationic polymer a candidate for treatment of mosquito bites.

In an embodiment of the polycationic polymer according to the invention, the polycationic polymer is for use as a topical skin composition, such as a cream or ointment.

For treating microbial infections and/or the above-mentioned examples, the polymer is preferably used as an ingredient for a topical skin composition, for application to the skin.

The object of the invention is further achieved with a composition, comprising a polymer according to the invention dissolved in a solvent, which solvent preferably is selected from an alcohol, water and/or a mixture thereof.

The solvent assists in forming a layer of the polymer on the surface on which the composition is being applied. This solvent may be volatile, such that it will readily evaporate after application, leaving the polymer on the surface.

In an embodiment of the composition according to the invention, the amount of polymer dissolved in the solvent is between 0,5 and 20 weight percent of the composition.

The provision of the polymer in such an amount makes it possible to come to a composition which is easy to apply. When applied at an amount below 0,5 weight percent of the composition, the amount is too little to be effective and/or to form a homogeneous layer after evaporation of the solvent.

In an embodiment of the composition according to the invention, the composition further comprises a polymer comprising a quaternary ammonium moiety, dissolved in the composition.

Quaternary ammonium moieties are known moieties for counteracting microbes such as bacteria. These moieties may therefore be advantageously used in a composition as a part of the polymer according to the invention. Since these have a specific way of acting on microbes, such a combination results in a synergistic effect, greater in size than the sum of each of the respective ingredients. This moiety may be part of a repeating unit of the polymer.

In a specific embodiment, the polymer comprising the quaternary ammonium moiety may be the polycationic polymer according to the invention, also comprising the repeating unit C. Alternatively or additionally, the quaternary ammonium moiety may be provided in the composition, not as a part of a polymer.

An aspect of the invention relates to the composition of the invention for use as a medicament.

An aspect of the invention relates to the composition of the invention for use as a topical skin composition, such as a cream or ointment.

The object of the invention is further achieved with a product, comprising a functionalized surface, comprising a layer of the polycationic polymer according to the invention and/or a composition according to the invention, on the functionalized surface.

As mentioned, the polymer according to the invention may be used for preventing growth of microbes on a surface. Such surfaces may for instance be body implants, but may also comprise surfaces prone to microbes and touched by humans, such as tables, door handles and shopping carts. The polymer may is coupled to the functionalized surface, preferably by physical coupling, although chemical coupling is also possible.

An aspect of the invention is the polycationic polymer according to the invention or the composition according to the invention, for use in the treatment or prevention of growth of microbes on a surface in a subject such as a human patient.

An embodiment is the polycationic polymer according to the invention or the composition according to the invention, for use in the treatment or prevention of growth of microbes on a surface in said subject, wherein the surface is the surface of a body implant.

In an embodiment of the product according to the invention, the product is a wound dressing.

The efficacy of the polymer according to the invention for the treatment of microbial infections makes the polymer and/or a composition comprising the polymer a suitable candidate for use in a wound dressing such as a bandage and patches.

### EXAMPLES

The following examples are provided to further illustrate the present invention and is not to be construed as limiting the invention in any manner.

### Materials

L-arginine hydrochloride methyl ester methacrylamide was prepared according to a method such as disclosed in paragraphs 3.2.1 to 3.2.3 of the MSc thesis "Synthesis and RAFT Polymerization of Arginine Containing Monomer To Investigate The Cell Membrane Translocation" by D. Ugur, for Izmir Institute of Technology, published in July 2014. The other materials were purchased from chemical suppliers.

### Example 1

A polycationic polymer according to the invention was prepared by dissolving 20 grams of L-arginine hydrochloride methyl ester methacrylamide with 4 grams of benzophenone methacrylate (BPMA), 40 grams of 2-ethylhexyl acrylate (2-EHA), 136 grams of butyl acrylate (BA) in 150 grams of methanol. This mixture was added to a reactor, together with1 gram of 2-2'-Azobis(2,4-dimethylvaleronitrile), dissolved in 10 grams of methanol. The contents of the reactor were inertized with nitrogen for 30 minutes, and subsequently, the mixture in the reactor was heated to 62 °C to start polymerization, which was carried out for 24 hours.

The polymer was isolated from the monomers and the solvent by drying with a rotavap at a temperature of 130°C at vacuum, at which temperature the polymer is liquid. The polymer obtained was applied to a surface by hot melt coating.

### Example 2

A polycationic polymer according to the invention was prepared by dissolving 8 grams of L-arginine hydrochloride methyl ester methacrylamide with 4 grams of methoxypoly(ethyleneglycol) methacrylate (MPEGMA) with a molecular weight of the PEG block of 350 grams per mole, 14 grams of methyl methacrylate (MMA), 14 grams of butyl acrylate (BA) in 40 grams of ethanol. This mixture was added to a reactor, together with 0,5 grams of 2-2'-Azobis(2,4-dimethylvaleronitrile), dissolved in 5 grams of ethanol. The contents of the reactor were inertized with nitrogen for 30 minutes, and subsequently, the mixture in the reactor was heated to 62°C to start polymerization. After 3 hours of polymerization, 0,5 grams of 2-2'-Azobis(2,4-dimethylvaleronitrile) dissolved in 5 grams of ethanol was added to the reactor to maximize conversion. Polymerization was carried out for 24 hours in total. The polymer was applied to a surface, and the solvent was removed by evaporation to the air, obtaining a polymeric film.

### Example 3

A polycationic polymer according to the invention was prepared by dissolving 10 grams of L-arginine hydrochloride methyl ester methacrylamide with 10 grams of methoxypoly(ethyleneglycol) methacrylate (MPEGMA) with a molecular weight of the PEG block of 350 grams per mole, 40 grams of butyl acrylate (BA) and 40 grams of methyl methacrylate (MMA) in 100 grams of ethanol. This mixture was added to the reactor, together with 1 gram of 2-2'-Azobis(2,4-dimethylvaleronitrile), dissolved in 10 grams of ethanol. The contents of the reactor were inertized with nitrogen for 30 minutes, and subsequently, the mixture in the reactor was heated to 62°C to start polymerization. After 3 hours of polymerization, 1 grams of 2-2'-Azobis(2,4-dimethylvaleronitrile) dissolved in 10 grams of ethanol was added to the reactor to maximize conversion. Polymerization was carried out for 24 hours in total.

The polymer was applied to a surface, and the solvent was removed by evaporation to the air, obtaining a polymeric film, which was tested according to ASTM E2149-13a. In this test, the polymer was shown to have an efficacy of 99,9%.

## Claims

1. Polycationic polymer, comprising a cationic repeating unit C, comprising a positively-charged protonated guanidine group, and a negatively-charged counterion, balancing the positive charge of this guanidine group, wherein the dispersity (M_{w}/Mₙ) of the polymer is 1,5 or higher determined according to the method mentioned in the specification, wherein the polycationic polymer comprises one or more than one further repeating unit, formed from a comonomer comprising a polymerizable group, wherein said at least one comonomer is a crosslinkable monomer, wherein the crosslinkable monomer is selected from benzophenone methacrylate (BPMA), benzophenone acrylate (BPA), a bifunctional acrylate or methacrylate, a trifunctional acrylate or methacrylate and a glycidyl acrylate or methacrylate.

2. Polycationic polymer according to claim 1, wherein the counterion is inorganic and preferably is selected from a chloride, bromide, iodide, fluoride, and more preferably is chloride.

3. Polycationic polymer according to claim 2, wherein the counterion is bound to the hydrogen by which the guanidine group is protonated.

4. Polycationic polymer according to any of the preceding claims, wherein said guanidine group is a terminal group.

5. Polycationic polymer according to any of the preceding claims, wherein said guanidine group has the following structure: 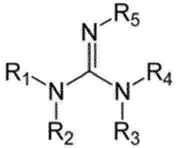
wherein one of R₁, R₂, R₃, R₄ and R₅ is an alkyl linking group, linking the guanidine group to the polymer backbone, and
wherein the others of R₁, R₂, R₃, R₄ and R₅ is H.

6. Polycationic polymer according to any of the preceding claims, wherein the cationic repeating unit further comprises an alkyl ester group.

7. Polycationic polymer according to any of the preceding claims, wherein the cationic repeating unit is formed from a monomer having a polymerizable group selected from an acrylate and a methacrylate.

8. Polycationic polymer according to claim 6 and 7, wherein the monomer has the following structure: 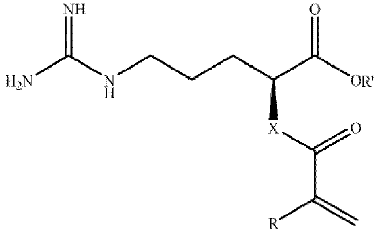
wherein X is NH, NH-alkyl-O, NH-alkyl-NR, NHCHzCH(OH)CHzO or NH-alkyl;
wherein each R is independently H or CH₃, and
wherein R' is alkyl.

9. Polycationic polymer according to claim 8, wherein R' is selected from methyl, ethyl, butyl and propyl and preferably is selected from methyl and ethyl.

10. Polycationic polymer according to any of the preceding claims, wherein at least one cationic repeating unit comprises at least one further guanidine group, wherein said further guanidine group is linked to said guanidine group via at least one saturated C-C bond.

11. Polycationic polymer according to any of the preceding claims, wherein the polymerizable group of said further comonomer is selected from an acrylate and a methacrylate.

12. Polycationic polymer according to any of the preceding claims, wherein the polymer is at least partly crosslinked.

13. Polycationic polymer according to claim 12, wherein the average degree of crosslinking of the polymer is between 0,05 and 3 mole percent.

14. Polycationic polymer according to any of the preceding claims, wherein at least one further comonomer is selected from:
a) at least one of benzyl methacrylate, butyl methacrylate, isobutyl acrylate, methyl methacrylate, ethyl methacrylate, propyl methacrylate, hexyl methacrylate, 2-ethylhexyl methacrylate, octyl methacrylate, lauryl methacrylate, stearyl methacrylate, p-tolyl methacrylate, sorbyl methacrylate, isobornyl methacrylate, benzyl acrylate, butyl acrylate, isobutyl acrylate, methyl acrylate, ethyl acrylate, propyl acrylate, hexyl acrylate, 2-ethylhexyl acrylate, octyl acrylate, lauryl acrylate, stearyl acrylate, p-tolyl acrylate, sorbyl acrylate, isobornyl acrylate, and/or
b) at least one of tri(ethylene glycol) methyl ether acrylate, 2-(2ethoxyethoxy)ethyl acrylate (EOEOEA), ethylene glycol methyl ether acrylate, 2-ethoxyethyl acrylate, di(ethylene glycol) methyl ether acrylate, hydroxyethyl acrylate, methoxypoly(ethyleneglycol) acrylate, ethylene glycol methyl ether methacrylate, 2-ethoxyethyl methacrylate, di(ethylene glycol) methyl ether methacrylate, tri(ethylene glycol) methyl ether methacrylate, 2-(2ethoxyethoxy)ethyl methacrylate (EOEOEMA), hydroxyethyl methacrylate, methoxypoly(ethyleneglycol) methacrylate
and mixtures thereof.

15. Polycationic polymer according to any of the claims 10 to 14, wherein the repeating unit C content of the polymer is between 1 and 50 percent by weight of the polymer, more preferably between 2 and 25 percent by weight of the polymer, most preferably between 5 and 15 percent by weight of the polymer, in particular approximately 10 percent by weight of the polymer.

16. Polycationic polymer according to any of the preceding claims, wherein the weight average molecular weight (M_{w}) of the polymer is between 10.000 and 1.000.000 Da, more preferably between 10.000 and 100.000 Da and wherein the weight average molecular weight (M_{w}) is determined according to the method mentioned in the specification.

17. Polycationic polymer according to any of the preceding claims, wherein the dispersity of the polymer is 6,0 or lower determined according to the method mentioned in the specification.

18. Polycationic polymer according to any of the preceding claims, for use as a medicament.

19. Product, comprising a functionalized surface, comprising a layer of the polycationic polymer according to any of claims 1 to 18.

20. Product according to claim 19, wherein the product is a wound dressing.

## Patentansprüche

1. Polykationisches Polymer, umfassend eine kationische Wiederholungseinheit C, die eine positiv geladene protonierte Guanidingruppe umfasst, und ein negativ geladenes Gegenion, das die positive Ladung dieser Guanidingruppe ausgleicht, wobei die Dispersität (M_{w}/Mₙ) des Polymers 1,5 oder höher ist, bestimmt nach dem in der Beschreibung genannten Verfahren, wobei das polykationische Polymer eine oder mehr als eine weitere Wiederholungseinheit umfasst, gebildet aus einem Comonomer, das eine polymerisierbare Gruppe umfasst, wobei das mindestens eine Comonomer ein vernetzbares Monomer ist, wobei das vernetzbare Monomer ausgewählt ist aus Benzophenonmethacrylat (BPMA), Benzophenonacrylat (BPA), einem bifunktionellen Acrylat oder Methacrylat, einem trifunktionellen Acrylat oder Methacrylat und einem Glycidylacrylat oder -methacrylat.

2. Polykationisches Polymer nach Anspruch 1, wobei das Gegenion anorganisch ist und vorzugsweise aus einem Chlorid, Bromid, Iodid, Fluorid ausgewählt ist und bevorzugter Chlorid ist.

3. Polykationisches Polymer nach Anspruch 2, wobei das Gegenion an den Wasserstoff gebunden ist, durch den die Guanidingruppe protoniert ist.

4. Polykationisches Polymer nach einem der voranstehenden Ansprüche, wobei die Guanidingruppe eine endständige Gruppe ist.

5. Polykationisches Polymer nach einem der voranstehenden Ansprüche, wobei die Guanidingruppe die folgende Struktur aufweist: 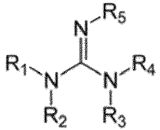
wobei einer von R₁, R₂, R₃, R₄ und R₅ eine Alkyl-Verbindungsgruppe ist, die die Guanidin-Gruppe mit dem Polymergerüst verbindet, und
wobei die anderen von R₁, R₂, R₃, R₄ und R₅ H sind.

6. Polykationisches Polymer nach einem der voranstehenden Ansprüche, wobei die kationische Wiederholungseinheit weiterhin eine Alkylestergruppe umfasst.

7. Polykationisches Polymer nach einem der voranstehenden Ansprüche, wobei die kationische Wiederholungseinheit aus einem Monomer mit einer polymerisierbaren Gruppe, ausgewählt aus einem Acrylat und einem Methacrylat, gebildet ist.

8. Polykationisches Polymer nach Anspruch 6 und 7, wobei das Monomer die folgende Struktur aufweist: 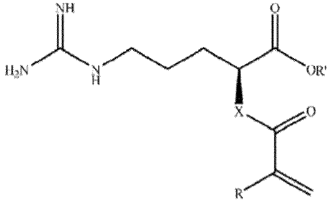
wobei X NH, NH-Alkyl-O, NH-Alkyl-NR, NHCH₂CH(OH)CH₂O oder NH-Alkyl ist;
wobei jedes R unabhängig voneinander H oder CH₃ ist; und
wobei R' Alkyl ist.

9. Polykationisches Polymer nach Anspruch 8, wobei R' ausgewählt ist aus Methyl, Ethyl, Butyl und Propyl und vorzugsweise ausgewählt ist aus Methyl und Ethyl.

10. Polykationisches Polymer nach einem der voranstehenden Ansprüche, wobei mindestens eine kationische Wiederholungseinheit mindestens eine weitere Guanidingruppe umfasst, wobei die weitere Guanidingruppe mit der Guanidingruppe über mindestens eine gesättigte C-C-Bindung verbunden ist.

11. Polykationisches Polymer nach einem der voranstehenden Ansprüche, wobei die polymerisierbare Gruppe des weiteren Comonomers ausgewählt ist aus einem Acrylat und einem Methacrylat.

12. Polykationisches Polymer nach einem der voranstehenden Ansprüche, wobei das Polymer zumindest teilweise vernetzt ist.

13. Polykationisches Polymer nach Anspruch 12, wobei der durchschnittliche Vernetzungsgrad des Polymers zwischen 0,05 und 3 Molprozent liegt.

14. Polykationisches Polymer nach einem der voranstehenden Ansprüche, wobei mindestens ein weiteres Comonomer ausgewählt ist aus:
a) mindestens einem von Benzylmethacrylat, Butylmethacrylat, Isobutylacrylat, Methylmethacrylat, Ethylmethacrylat, Propylmethacrylat, Hexylmethacrylat, 2-Ethylhexylmethacrylat, Octylmethacrylat, Laurylmethacrylat, Stearylmethacrylat, p-Tolylmethacrylat, Sorbylmethacrylat, Isobornylmethacrylat, Benzylacrylat, Butylacrylat, Isobutylacrylat, Methylacrylat, Ethylacrylat, Propylacrylat, Hexylacrylat, 2-Ethylhexylacrylat, Octylacrylat, Laurylacrylat, Stearylacrylat, p-Tolylacrylat, Sorbylacrylat, Isobornylacrylat, und/oder
b) mindestens einem von Tri(ethylenglykol)methyletheracrylat, 2-(2-Ethoxy-ethoxy)ethylacrylat (EOEOEA), Ethylenglykolmethyletheracrylat, 2-Ethoxyethylacrylat, Di(ethylenglykol)methyletheracrylat, Hydroxyethylacrylat, Methoxypoly(ethylenglykol)acrylat, Ethylenglykolmethylethermethacrylat, 2-Ethoxyethylmethacrylat, Di(ethylenglykol)methylethermethacrylat, Tri(ethylenglykol)methylethermethacrylat, 2-(2-Ethoxyethoxy)ethylmethacrylat (EOEOEMA), Hydroxyethylmethacrylat, Methoxypoly(ethylenglykol)methacrylat und deren Mischungen.

15. Polykationisches Polymer nach einem der Ansprüche 10 bis 14, wobei der Gehalt an Wiederholungseinheit C des Polymers zwischen 1 und 50 Gewichtsprozent des Polymers beträgt, bevorzugter zwischen 2 und 25 Gewichtsprozent des Polymers, am meisten bevorzugt zwischen 5 und 15 Gewichtsprozent des Polymers, insbesondere etwa 10 Gewichtsprozent des Polymers.

16. Polykationisches Polymer nach einem der voranstehenden Ansprüche, wobei das gewichtsmittlere Molekulargewicht (M_{w}) des Polymers zwischen 10.000 und 1.000.000 Da, bevorzugter zwischen 10.000 und 100.000 Da liegt und wobei das gewichtsmittlere Molekulargewicht (M_{w}) nach dem in der Beschreibung genannten Verfahren bestimmt wird.

17. Polykationisches Polymer nach einem der voranstehenden Ansprüche, wobei die Dispersität des Polymers 6,0 oder weniger beträgt, bestimmt nach dem in der Beschreibung genannten Verfahren.

18. Polykationisches Polymer nach einem der voranstehenden Ansprüche, zur Anwendung als Medikament.

19. Produkt, umfassend eine funktionalisierte Oberfläche, die eine Schicht des polykationischen Polymers nach einem der Ansprüche 1 bis 18 umfasst.

20. Produkt nach Anspruch 19, wobei das Produkt ein Wundverband ist.

## Revendications

1. Polymère polycationique, comprenant une unité de répétition cationique C, comprenant un groupe guanidine protoné chargé positivement, et un contre-ion chargé négativement, équilibrant la charge positive de ce groupe guanidine, où la dispersité (M_{w}/Mₙ) du polymère est de 1,5 ou plus déterminée selon le procédé mentionné dans la spécification, où le polymère polycationique comprend une ou plus d'une unité de répétition supplémentaire, formée à partir d'un comonomère comprenant un groupe polymérisable, où ledit au moins un comonomère est un monomère réticulable, où le monomère réticulable est sélectionné parmi le méthacrylate de benzophénone (BPMA), l'acrylate de benzophénone (BPA), un acrylate ou méthacrylate bifonctionnel, un acrylate ou méthacrylate trifonctionnel et un acrylate ou méthacrylate de glycidyle.

2. Polymère polycationique selon la revendication 1, où le contre-ion est inorganique et est de préférence sélectionné parmi un chlorure, un bromure, un iodure, un fluorure, et plus préférablement un chlorure.

3. Polymère polycationique selon la revendication 2, où le contre-ion est lié à l'hydrogène par lequel le groupe guanidine est protoné.

4. Polymère polycationique selon l'une quelconque des revendications précédentes, où ledit groupe guanidine est un groupe terminal.

5. Polymère polycationique selon l'une quelconque des revendications précédentes, où ledit groupe guanidine a la structure suivante : 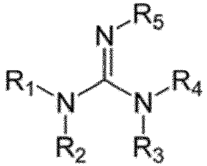
où l'un des R₁, R₂, R₃, R₄ et R₅ est un groupe de liaison alkyle, reliant le groupe guanidine au squelette du polymère, et
où les autres de R₁, R₂, R₃, R₄ et R₅ est H.

6. Polymère polycationique selon l'une quelconque des revendications précédentes, où l'unité de répétition cationique comprend en outre un groupe ester d'alkyle.

7. Polymère polycationique selon l'une quelconque des revendications précédentes, où l'unité de répétition cationique est formée à partir d'un monomère ayant un groupe polymérisable sélectionné parmi un acrylate et un méthacrylate.

8. Polymère polycationique selon les revendications 6 et 7, où le monomère a la structure suivante : 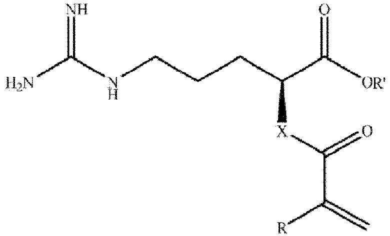
où X est NH, NH-alkyl-O, NH-alkyl-NR, NHCH₂CH(OH)CH₂O ou NH-alkyl ;
où chaque R est indépendamment H ou CH₃, et
où R' est alkyle.

9. Polymère polycationique selon la revendication 8, où R' est sélectionné parmi méthyle, éthyle, butyle et propyle et de préférence est sélectionné parmi méthyle et éthyle.

10. Polymère polycationique selon l'une quelconque des revendications précédentes, où au moins une unité répétitive cationique comprend au moins un groupe guanidine supplémentaire, où ledit groupe guanidine supplémentaire est lié audit groupe guanidine via au moins une liaison C-C saturée.

11. Polymère polycationique selon l'une quelconque des revendications précédentes, où le groupe polymérisable dudit comonomère supplémentaire est sélectionné parmi un acrylate et un méthacrylate.

12. Polymère polycationique selon l'une quelconque des revendications précédentes, où le polymère est au moins partiellement réticulé.

13. Polymère polycationique selon la revendication 12, où le degré moyen de réticulation du polymère est entre 0,05 et 3 mole pour cent.

14. Polymère polycationique selon l'une quelconque des revendications précédentes, où au moins un comonomère supplémentaire est sélectionné parmi
a) au moins l'un parmi méthacrylate de benzyle, méthacrylate de butyle, acrylate d'isobutyle, méthacrylate de méthyle, méthacrylate d'éthyle, méthacrylate de propyle, méthacrylate d'hexyle, méthacrylate de 2-éthylhexyle, méthacrylate d'octyle, méthacrylate de lauryle, méthacrylate de stéaryle, méthacrylate de p-tolyle, méthacrylate de sorbyle, méthacrylate d'isobornyle, acrylate de benzyle, acrylate de butyle, acrylate d'isobutyle, acrylate de méthyle, acrylate d'éthyle, acrylate de propyle, acrylate d'hexyle, acrylate de 2-éthylhexyle, acrylate d'octyle, acrylate de lauryle, acrylate de stéaryle, acrylate de p-tolyle, acrylate de sorbyle, acrylate d'isobornyle, et/ou
b) au moins l'un parmi acrylate d'éther méthylique de tri(éthylène glycol), acrylate de 2-(2éthoxyéthoxy)éthyle (EOEOEA), acrylate d'éther méthylique d'éthylène glycol, acrylate de 2-éthoxyéthyle, acrylate d'éther méthylique de di(éthylène glycol), acrylate d'hydroxyéthyle, acrylate de méthoxypoly(éthylène glycol), méthacrylate d'éther méthylique d'éthylène glycol, méthacrylate de 2-éthoxyéthyle, méthacrylate d'éther méthylique de di(éthylène glycol), méthacrylate d'éther méthylique de tri(éthylène glycol), méthacrylate de 2-(2éthoxyéthoxy)éthyle (EOEOEMA), méthacrylate d'hydroxyéthyle, méthacrylate de méthoxypoly(éthylène glycol) et des mélanges de ceux-ci.

15. Polymère polycationique selon l'une quelconque des revendications 10 à 14, où le contenu de l'unité de répétition C du polymère est compris entre 1 et 50 pour cent en poids du polymère, plus préférablement entre 2 et 25 pour cent en poids du polymère, le plus préférablement entre 5 et 15 pour cent en poids du polymère, en particulier approximativement 10 pour cent en poids du polymère.

16. Polymère polycationique selon l'une quelconque des revendications précédentes, où le poids moléculaire moyen en poids (M_{w}) du polymère est entre 10 000 et 1 000 000 Da, plus préférablement entre 10 000 et 100 000 Da et où le poids moléculaire moyen en poids (M_{w}) est déterminé selon le procédé mentionné dans la spécification.

17. Polymère polycationique selon l'une quelconque des revendications précédentes, où la dispersité du polymère est de 6,0 ou inférieure déterminée selon le procédé mentionné dans la spécification.

18. Polymère polycationique selon l'une quelconque des revendications précédentes, pour utilisation comme un médicament.

19. Produit, comprenant une surface fonctionnalisée, comprenant une couche du polymère polycationique selon l'une quelconque des revendications 1 à 18.

20. Produit selon la revendication 19, où le produit est un pansement.
